# EUROPEAN PATENT APPLICATION

(11) **EP 4 350 346 A1**
(43) Date of publication of application: **10.04.2024**
(21) Application number: 22811029.2
(22) Date of filing: 31.03.2022
(51) Int. Cl.: G01N 33/30

(54) **MONITORING AND DIAGNOSING DEVICE, MONITORING AND DIAGNOSING SYSTEM, AND MONITORING AND DIAGNOSING METHOD**

(30) Priority: 27.05.2021 JP 2021089204
(71) Applicant: Moresco Corporation, Kobe-shi, Hyogo 650-0047 (JP)
(72) Inventor: KUSUMOTO Kazuki, Kobe-shi Hyogo 650-0047 (JP)
(74) Representative: Viering, Jentschura & Partner mbB Patent- und Rechtsanwälte
(86) International application number: PCT/JP2022/016545
(87) International publication number: WO 2022/249755

(57) **Abstract**

A monitoring and diagnosing apparatus 11 monitors a property of water-soluble lubricating oil circulating in a working machine 13 and diagnoses the property of the water-soluble lubricating oil. The monitoring and diagnosing apparatus 11 includes a first control unit 15 that controls the monitoring and diagnosing apparatus 11 and a detection unit 16 that detects the property of the water-soluble lubricating oil circulating in the working machine 13. The first control unit 15 has a detected data acquisition section 61 that acquires detected data detected by the detection unit 16, and a diagnosis section 62 that diagnoses the property of the water-soluble lubricating oil based on the detected data acquired by the detected data acquisition section 61.

## Description

### [Technical Field]

This invention relates to a monitoring and diagnosing apparatus, a monitoring and diagnosing system, and a monitoring and diagnosing method. This application claims the benefit of priority to Japanese Patent Application No. 2021-89204, filed May 27, 2021, the entire contents of which is incorporated herein by reference in its entirety.

### [Background Art]

Systems for diagnosing the properties of oil in working machines are disclosed (see, for example, PTL 1, PTL 2, and PTL 3).

### [Citation List]

### [Patent Literature]

[PTL 1] JP-A-2016-113819
[PTL 2] WO 2019/021502
[PTL 3] JP-A-H02-145966

### [Summary of Invention]

### [Technical Problem]

Water-containing hydraulic oils (water-soluble lubricating oils), represented by water/glycol-based hydraulic oils, are sometimes used as hydraulic oils. The composition of such water-soluble lubricating oils differs significantly from that of mineral oil-based hydraulic oils because of the water content. For stable operation of hydraulic apparatuses, it is also required to properly understand the degree of deterioration, service life, or the like of water-soluble lubricating oils and perform efficient maintenance. In other words, the properties of water-soluble lubricating oils are required to be efficiently monitored to diagnose the property of water-soluble lubricating oils. The technologies disclosed in PTL 1, PTL 2, and PTL 3 mentioned above are targeted at mineral oil-based hydraulic oils, and it is difficult to apply them to water-soluble lubricating oils.

Therefore, one of the objectives is to provide a monitoring and diagnosing apparatus that can efficiently monitor the property of water-soluble lubricating oil and diagnose the property of water-soluble lubricating oil.

### [Solution to Problem]

A monitoring and diagnosing apparatus according to the present invention monitors a property of water-soluble lubricating oil circulating in a working machine and diagnoses the property of the water-soluble lubricating oil. The monitoring and diagnosing apparatus includes a first control unit that controls the monitoring and diagnosing apparatus and a detection unit that detects the property of the water-soluble lubricating oil circulating in the working machine. The first control unit includes a detected data acquisition section that acquires detected data detected by the detection unit and a diagnosis section that diagnoses the property of the water-soluble lubricating oil based on the detected data acquired by the detected data acquisition section.

### [Effects of Invention]

According to the above monitoring and diagnosing apparatus, the property of water-soluble lubricating oil can be efficiently monitored, and the property of water-soluble lubricating oil can be diagnosed.

### [Brief Description of Drawings]

FIG. 1 is a schematic diagram schematically illustrating a monitoring and diagnosing system including a monitoring and diagnosing apparatus in a first embodiment, which is one embodiment of the present invention.
FIG. 2 is a block diagram illustrating the constitution of a first control unit included in the monitoring and diagnosing apparatus of the first embodiment illustrated in FIG. 1.
FIG. 3 is a block diagram of a portion of the monitoring and diagnosing system.
FIG. 4 is a flowchart of typical steps of a monitoring and diagnosing method when water-soluble lubricating oil is monitored and diagnosed using the monitoring and diagnosing system including the monitoring and diagnosing apparatus in the first embodiment.
FIG. 5 is a flowchart illustrating an example of typical steps in the diagnosing step.
FIG. 6 is a graph illustrating an example of the relationship between water content and elapsed time.
FIG. 7 is a graph illustrating an example of the relationship between kinematic viscosity at 40°C and elapsed time.
FIG. 8 is a schematic diagram illustrating an example of a display screen in a display displaying the diagnosis results of the monitoring and diagnosing apparatus.

### [Description of Embodiments]

### [Summary of Embodiments]

A monitoring and diagnosing apparatus according to the present invention monitors the property of water-soluble lubricating oil circulating in a working machine and diagnoses the property of the water-soluble lubricating oil. The monitoring and diagnosing apparatus includes a first control unit that controls the monitoring and diagnosing apparatus and a detection unit that detects the property of the water-soluble lubricating oil circulating in the working machine. The first control unit includes a detected data acquisition section that acquires detected data detected by the detection unit and a diagnosis section that diagnoses the property of the water-soluble lubricating oil based on the detected data acquired by the detected data acquisition section.

According to the monitoring and diagnosing apparatus of the present invention, in a working machine using water-soluble lubricating oil, the property of the water-soluble lubricating oil are detected by the detection unit, and the property of the water-soluble lubricating oil are diagnosed by the diagnosis section. This would reduce the need for periodic sampling and analysis of the water-soluble lubricating oil by human hands in terms of maintenance and long-term management. Thus, according to the above monitoring and diagnosing apparatus, the property of water-soluble lubricating oil can be efficiently monitored, and the property of water-soluble lubricating oil can be diagnosed.

In the monitoring and diagnosing apparatus, the detection unit may detect at least one of the water content of the water-soluble lubricating oil, the temperature of the water-soluble lubricating oil, the density of the water-soluble lubricating oil, the viscosity of the water-soluble lubricating oil, the hydrogen ion concentration of the water-soluble lubricating oil, the cleanliness of the water-soluble lubricating oil, and the appearance of the water-soluble lubricating oil. In this way, the property of the water-soluble lubricating oil can be checked by detecting the above parameter, and the property of the water-soluble lubricating oil can be diagnosed in a more detailed and comprehensive manner.

In the above monitoring and diagnosing apparatus, the detection unit may detect water content. Although the way of measuring water content is not limited, the detection unit may detect a refractive index of the water-soluble lubricating oil, and derive the water content of the water-soluble lubricating oil based on the detected refractive index of the water-soluble lubricating oil to detect the water content of the water-soluble lubricating oil. In this way, the water content of water-soluble lubricating oil, an important parameter in water-soluble lubricating oils, can be detected more accurately and simply. Thus, more efficient monitoring and diagnosis of the property of water-soluble lubricating oil can be performed.

In the monitoring and diagnosing apparatus, the detection unit may detect the viscosity of the water-soluble lubricating oil, the density of the water-soluble lubricating oil, and the temperature of soluble lubricant and derive the kinematic viscosity of the water-soluble lubricating oil at a given temperature. In this way, the kinematic viscosity of the water-soluble lubricating oil at a given temperature can be derived properly.

In the monitoring and diagnosing apparatus, the detection unit may detect the water content of the water-soluble lubricating oil, the viscosity of the water-soluble lubricating oil, the density of the water-soluble lubricating oil, and the temperature of the water-soluble lubricating oil and calculate the kinematic viscosity at a given temperature using the viscosity, the density, and the temperature. The diagnosis section may estimate the hypothetical kinematic viscosity of the water-soluble lubricating oil at a given temperature when the water-soluble lubricating oil is at an appropriate water content based on the water content detected by the detection unit, the kinematic viscosity at the given temperature, and the standard correlation between appropriate water content and kinematic viscosity at the given temperature stored in advance and determine the degree of degradation of the water-soluble lubricating oil. In this way, the degree of deterioration of the water-soluble lubricating oil can be properly determined.

In the above monitoring and diagnosing apparatus, the diagnosis section may determine whether the detected data is outside a prescribed numerical range. The first control unit may further include a warning notification section that notifies a warning if the diagnosis section determines that the value is outside the prescribed numerical range. In this way, it becomes easier to make this warning known when it is necessary to issue a warning as a serious consequence of the diagnostic results. In this case, the user may be notified by at least one warning of a displayed warning, such as an alert on a screen, a voice warning, or a warning via a network, such as e-mails.

The monitoring and diagnosing system of the present invention is a monitoring and diagnosing system including the monitoring and diagnosing apparatus and includes a data storage unit that accumulates diagnostic data that are diagnostic results by the diagnosis section and/or the detected data and a second control unit that controls the diagnostic data and/or detected data accumulated in the data storage unit to be reflected in the next diagnosis by the diagnosis section. The diagnosis section diagnoses the property of the water-soluble lubricating oil based on the diagnostic data and/or detected data accumulated in the data storage unit. In this way, a diagnosis can be made more appropriately based on the accumulated diagnostic data.

The monitoring and diagnosing system may include at least either one of a water storage unit that stores water and a chemical liquid storage unit that stores a chemical liquid constituting the water-soluble lubricating oil. The first control unit includes a supply control section that controls the supply of at least either one of the water and the chemical liquid from at least either one of the water storage unit and the chemical liquid storage unit to the water-soluble lubricating oil in accordance with the diagnosis results by the diagnosis section. In this way, adjusting one of the properties of water-soluble lubricating oil, such as the water content of water-soluble lubricating oil, becomes easier without manual intervention. Thus, a more user-desired monitoring and diagnosing system can be provided. The chemical liquid is used to adjust the reverse alkalinity which indicate the amount of alkaline components, pH, kinematic viscosity, and the like, which indicate the amount of alkaline components. Reverse alkalinity is also called alkalinity value.

In the monitoring and diagnosing system, the supply control section may determine the supply amount of at least either one of the water and the chemical liquid to be added to the water-soluble lubricating oil based on the hypothetical kinematic viscosity estimated by the diagnosis section. In this way, at least either one of the water and the chemical liquid can be supplied in a more appropriate amount.

In the above monitoring and diagnosing system, the second control unit may control the first control unit to reflect the accumulated diagnosis data and/or the accumulated detected data in the next diagnosis. In this way, a more accurate diagnosis can be made based on the diagnosis data and detected data.

In the above monitoring and diagnosing system, the first control unit may further include an output control section that displays and outputs the diagnosis results by the diagnosis section. In this way, diagnostic results can be output visually. Thus, the diagnosis results can be communicated more clearly to the user.

The monitoring and diagnosing method according to the present invention monitors the property of water-soluble lubricating oil used in working machines and diagnoses the property of the water-soluble lubricating oil. The monitoring and diagnosing method includes the steps of: acquiring water-soluble lubricating oil from a working machine; detecting, after the step of acquiring the water-soluble lubricating oil, the property of the acquired water-soluble lubricating oil; acquiring, after the step of detecting the property of the water-soluble lubricating oil, data of the detected the property of the water-soluble lubricating oil; and diagnosing, after the step of acquiring the data of the property of the water-soluble lubricating oil, the property of the water-soluble lubricating oil based on the acquired data on the property of the water-soluble lubricating oil.

According to such a monitoring and diagnosing method, the property of water-soluble lubricating oil can be efficiently monitored, and the property of water-soluble lubricating oil can be diagnosed comprehensively.

### [Specific Examples of Embodiments]

Next, an example of specific embodiments of the monitoring and diagnosing apparatus of the present application will be described with reference to the drawings. In the following drawings, identical or equivalent parts are marked with the same reference number, and the explanation will not be repeated.

### (First Embodiment)

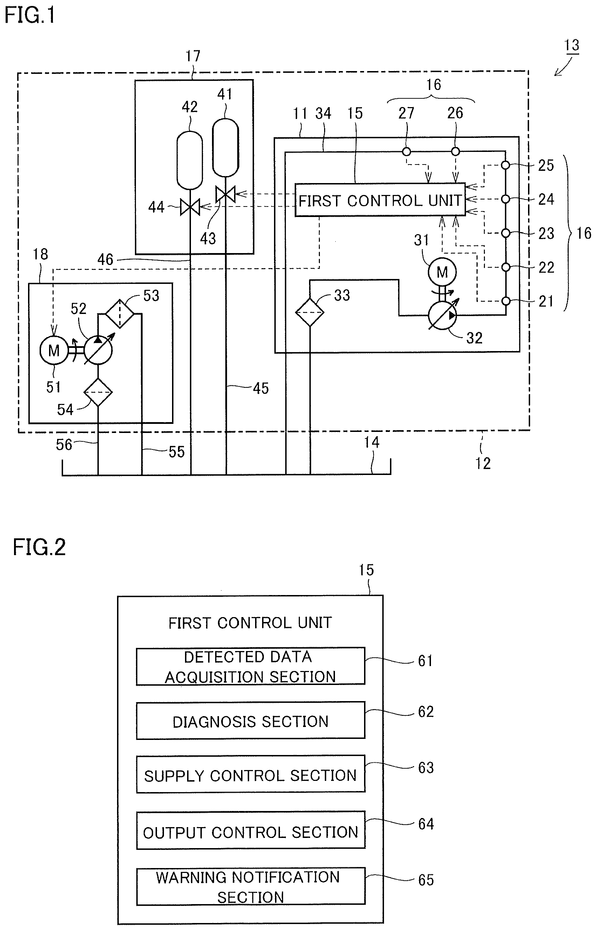

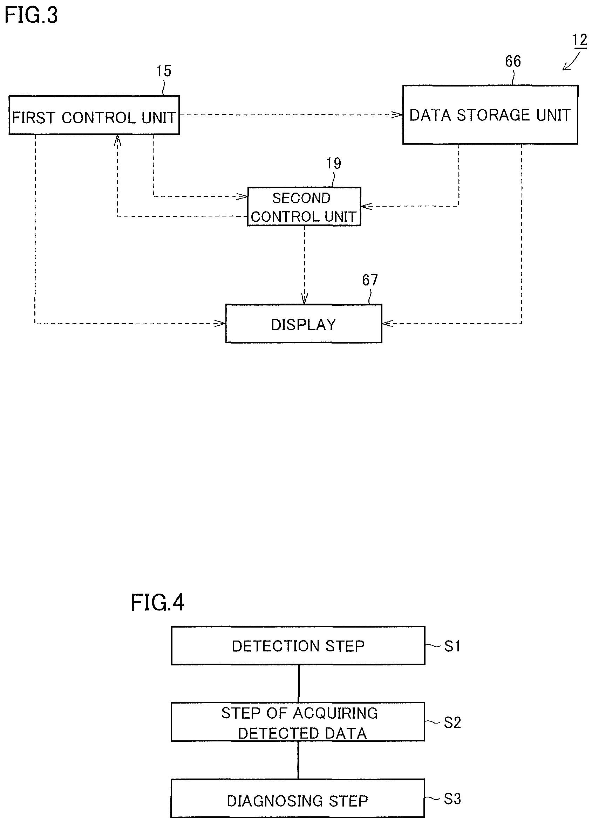

FIG. 1 is a schematic diagram schematically illustrating a monitoring and diagnosing system including a monitoring and diagnosing apparatus in the first embodiment, which is one embodiment of the present invention. FIG. 2 is a block diagram illustrating the constitution of a first control unit included in the monitoring and diagnosing apparatus of the first embodiment illustrated in FIG. 1. FIG. 3 is a block diagram of a portion of the monitoring and diagnosing system. Referring to FIGs. 1, 2, and 3, the monitoring and diagnosing apparatus 11 in the first embodiment is a monitoring and diagnosing apparatus that monitors the properties of water-soluble lubricating oil, including water/glycol-based hydraulic oil, used in a working machine 13, such as a hydraulic system and diagnoses the water-soluble lubricating oil. The monitoring and diagnosing apparatus 11 included in the monitoring and diagnosing system 12 is disposed, for example, in the working machine 13. In FIG. 1, the monitoring and diagnosing system 12 is indicated by a dash-dot-dash line. In the following description, as an example, the monitoring and diagnosing system 12 is constituted of the monitoring and diagnosing apparatus 11, the water and chemical liquid supply unit 17, and the filtration unit 18, but they may be mounted integrally in a single apparatus. Alternatively, the first control unit 15 and the second control unit 19 may be configured as a single unit.

The working machine 13 includes an oil tank 14 that temporarily stores water-soluble lubricating oil circulating in the working machine 13. The monitoring and diagnosing apparatus 11 monitors the properties of the water-soluble lubricating oil stored in the oil tank 14 and diagnoses the properties of the water-soluble lubricating oil.

The monitoring and diagnosing apparatus 11 includes a first control unit 15 that controls the monitoring and diagnosing apparatus 11 itself, a detection unit 16, a motor 31, a feed pump 32, and a filter 33. The monitoring and diagnosing apparatus 11 operates the feed pump 32 by the motor 31 to take some of the water-soluble lubricating oil from the oil tank 14 into the monitoring and diagnosing apparatus 11. In this case, some of the water-soluble lubricating oil is taken into the monitoring and diagnosing apparatus 11 using a pipe 34 that connects the monitoring and diagnosing apparatus 11 to the oil tank 14. At this time, the water-soluble lubricating oil that has passed through the filter 33 is used as the water-soluble lubricating oil for detection.

The detection unit 16 detects the properties of the water-soluble lubricating oil circulating in the working machine 13. Specifically, the detection unit 16 includes a refractive index sensor 21 that detects the refractive index of the water-soluble lubricating oil as data used to derive the water content of the water-soluble lubricating oil, a viscosity sensor 22 that detects the viscosity of the water-soluble lubricating oil, a density sensor 23 that detects the density of the water-soluble lubricating oil, a temperature sensor 24 that detects the temperature of the water-soluble lubricating oil, a cleanliness sensor 25 that detects the cleanliness of the water-soluble lubricating oil, a hydrogen ion concentration detection sensor 26 that detects the hydrogen ion concentration (pH) of the water-soluble lubricating oil, and a camera 27 that detects the appearance of the water-soluble lubricating oil. The refractive index sensor 21, viscosity sensor 22, density sensor 23, temperature sensor 24, cleanliness sensor 25, hydrogen ion concentration detection sensor 26, and camera 27 are arranged in order from upstream of the pipe 34. The monitoring and diagnosing apparatus 11 detects the properties of the water-soluble lubricating oil, such as the viscosity of the water-soluble lubricating oil, by the detection unit 16. After detection by detection unit 16, the water-soluble lubricating oil is returned to the oil tank 14 again through the pipe 34. The cleanliness sensor 25 is a particle counter, which, as an example, counts particles by shielding light illuminated on the lubricating oil to measure cleanliness. Appearance is detected by color tone or the like based on images captured by the camera 27.

The first control unit 15 includes a detected data acquisition section 61, a diagnosis section 62, a supply control section 63, an output control section 64, and a warning notification section 65. The detected data acquisition section 61 acquires detected data detected by the detection unit 16. The diagnosis section 62 diagnoses the properties of the water-soluble lubricating oil based on the detected data acquired by the detected data acquisition section 61. The output control section 64 displays and outputs the diagnosis results by the diagnosis section 62. The warning notification section 65 notifies a warning if the diagnosis section 62 determines that the detected data is outside a prescribed numerical range.

The monitoring and diagnosing system 12 including the monitoring and diagnosing apparatus 11 in the first embodiment includes a water and chemical liquid supply unit 17. The water and chemical liquid supply unit 17 includes a water tank 41 as a water storage unit to store water, a chemical liquid tank 42 as a chemical liquid storage unit to store a chemical liquid that constitutes the water-soluble lubricating oil, a solenoid valve 43 installed in the middle of the pipe 45 connecting the water tank 41 and the oil tank 14, and a solenoid valve 44 disposed in the middle of the pipe 46 connecting the chemical liquid tank 42 and the oil tank 14. Both solenoid valves 43 and 44 are opened or closed under the control of the first control unit 15. By opening the solenoid valve 43, water can be supplied to the oil tank 14 through the pipe 45. By opening solenoid valve 44, a chemical liquid can be supplied to the oil tank 14 through the pipe 46. The supply control section 63 controls the supply of water and the chemical liquid from the water tank 41 and chemical liquid tank 42 to the water-soluble lubricating oil according to the diagnosis results by the diagnosis section 62.

The monitoring and diagnosing system 12 including the monitoring and diagnosing apparatus 11 in the first embodiment includes a filtration unit 18. The filtration unit 18 includes a motor 51, a feed pump 52, and filters 53 and 54. The first control unit 15 can control the operation of the motor 51. By driving the motor 51 to operate the feed pump 52, some of the water-soluble lubricating oil can be fed from the oil tank 14 through the pipe 55 to the filter 53. The filter 53 allows the water-soluble lubricating oil fed from the oil tank 14 to be filtered to promote the removal of foreign matters actively. The filtered water-soluble lubricating oil is returned to the oil tank 14 again by the pipe 56 through the filter 54. The first control unit 15 controls the filtration unit 18 to filter the water-soluble lubricating oil according to the diagnosis results by the diagnosis section 62.

The configuration of the monitoring and diagnosing system 12 is described here. The monitoring and diagnosing system 12 includes a monitoring and diagnosing apparatus 11, a second control unit 19, a data storage unit 66, and a display 67. The data storage unit 66 accumulates diagnostic data, which are the diagnosis results by the diagnosis section 62. For example, a server or similar apparatus is used as the data storage unit 66. The second control unit 19 controls the diagnosis data accumulated in the data storage unit 66 to be reflected in the next diagnosis by the diagnosis section 62. In other words, the learning function in the second control unit 19 can be used. For example, the rate of change or trend of change in water content, pH, kinematic viscosity, and the like may be derived from the accumulated diagnostic data to predict the degree of deterioration of water-soluble lubricating oil and to inform the user of the timing of replacement or the like. The data storage unit 66 may consist of a plurality of memories. For example, the data storage unit 66 may exist separately in the first control unit 15 and the second control unit 19.

Next, a method of monitoring and diagnosing water-soluble lubricating oil circulating in a working machine 13 using a monitoring and diagnosing system 12 that includes the monitoring and diagnosing apparatus 11 in the first embodiment is described.

FIG. 4 is a flowchart of typical steps of a monitoring and diagnosing method when water-soluble lubricating oil is monitored and diagnosed using the monitoring and diagnosing system 12 including the monitoring and diagnosing apparatus 11 in the first embodiment. Referring to FIG. 4, in the monitoring and diagnosing method using the monitoring and diagnosing apparatus 11 in the first embodiment, a detection step is first implemented as a step (S1). In this step (S1), the water content, kinematic viscosity, density, temperature, cleanliness, pH, and appearance of the water-soluble lubricating oil are detected using the refractive index sensor 21, viscosity sensor 22, density sensor 23, temperature sensor 24, cleanliness sensor 25, hydrogen ion concentration detection sensor 26, and camera 27. In this case, these properties are detected inline, i.e., detected while the working machine 13 is running. Next, the step of acquiring detected data is performed as a step (S2). In this process (S2), data from each sensor in the detection unit 16 is acquired. Then, the diagnosing step is performed as a step (S3). Specifically, the diagnostic step is performed in the following manner.

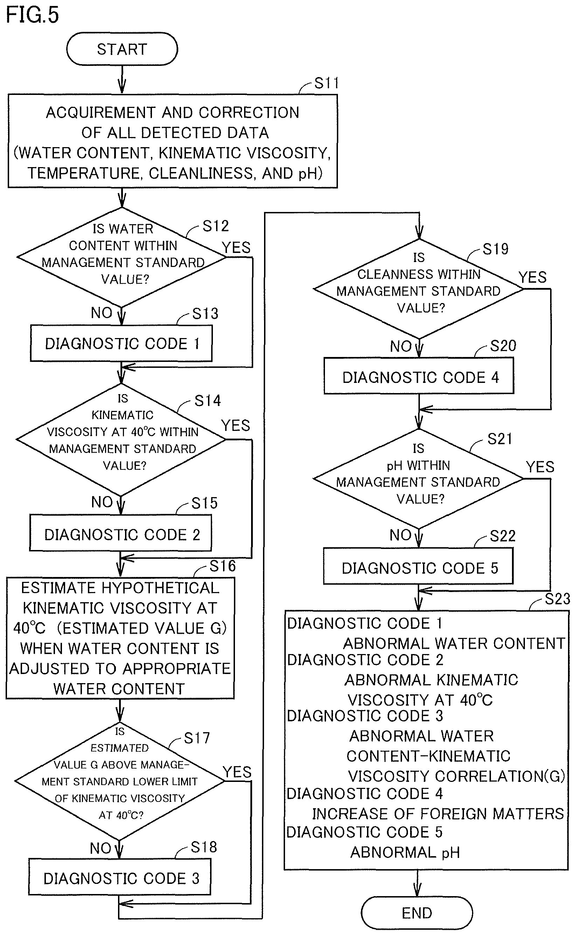

FIG. 5 is a flowchart illustrating an example of typical steps in the diagnosing step. Referring to FIG. 5, first, detected data detected by all sensors in the detection unit 16, including the refractive index sensor 21, are acquired and corrected. Detected data may be acquired continuously or at any intervals (for example, every 15 minutes, every hour, etc.). The water content is derived by performing a temperature correction to the refractive index of the water-soluble lubricating oil detected by the refractive index sensor 21. As for kinematic viscosity at 40°C, kinematic viscosity is derived from absolute viscosity and density, and the obtained kinematic viscosity is corrected for temperature to obtain a kinematic viscosity at 40°C. That is, corrected detected data of the water content, kinematic viscosity, temperature, cleanliness, and pH are acquired (S11). As an example, the kinematic viscosity at 40°C is derived here as a given temperature, but the given temperature can be set as appropriate.

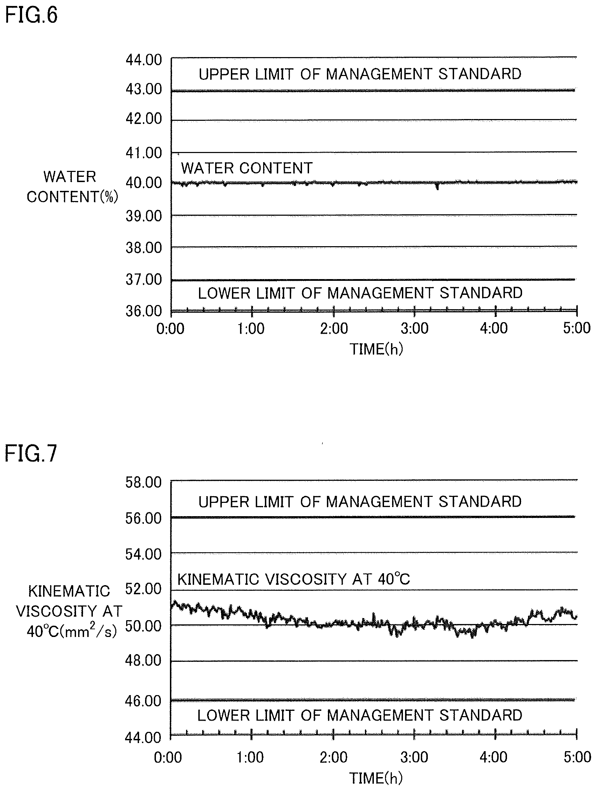

After that, the diagnosis section 62 determines whether the water content is within the management standard value (S12). FIG. 6 is a graph illustrating an example of the relationship between water content and elapsed time. In FIG. 6, the horizontal axis indicates the elapsed time (h (hours)), and the vertical axis indicates the water content (%). The graph shows the upper and lower limits of the management standard for water content. The upper and lower limits of the management standard for water content are each the values set depending on the type of water-soluble lubricating oil used. In the example illustrated in the graph in FIG. 6, the upper management standard limit is 43 (%), and the lower management standard limit is 37 (%). The change in water content over time is illustrated, for example, by the graph shown in FIG. 6.

If the water content is outside the management standard value (NO in S12), diagnostic code 1 is flagged (S13). The diagnostic code 1 is a flag that is raised when water-soluble lubricating oil deviates from the management standard range. This diagnostic code 1 and the diagnostic codes 2 to 5, which will be described below, are codes used on the program and are not displayed to the user. Then, the process proceeds to the next step. If the water content is within the management standard value (YES in S12), the process directly proceeds to the next step.

Next, whether the value of the kinematic viscosity at 40°C is within the management standard value is determined (S14). FIG. 7 is a graph illustrating an example of the relationship between kinematic viscosity at 40°C and elapsed time. In FIG. 7, the horizontal axis indicates the elapsed time (h (hours)), and the vertical axis indicates the kinematic viscosity at 40°C (mm²/s (seconds)). The graph shows the upper and lower limits of the management standard for kinematic viscosity at 40°C. The upper and lower limits of the management standard for kinematic viscosity at 40°C are each the values set depending on the type of water-soluble lubricating oil used. In the example illustrated in the graph in FIG. 7, the upper limit of the management standard is 56 (mm²/s), and the lower limit of the management standard is 46 (mm²/s). The change over time in kinematic viscosity at 40°C is illustrated, for example, in the graph shown in FIG. 7.

If the value of the kinematic viscosity at 40°C is outside the management standard value (NO in S14), the diagnostic code 2 is flagged (S15). The diagnostic code 2 is a flag that is raised when the kinematic viscosity at 40°C of water-soluble lubricating oil deviates from the set management standard range. Then, the process proceeds to the next step. If the value of the kinematic viscosity at 40°C is within the management standard value (YES in S14), the process directly proceeds to the next step.

Next, whether the water-soluble lubricating oil has the ability to maintain an appropriate viscosity is determined. Based on the water content and kinematic viscosity at 40°C obtained in S11 and the standard correlation between water content and kinematic viscosity stored in advance, the hypothetical kinematic viscosity at 40°C (estimated value G) when water is added to the water-soluble lubricating oil in use, and the water content is adjusted to the appropriate water content is estimated (S16). Then, whether the estimated value G is within the management standard range (for example, whether the estimated value G is above the lower limit of the management standard) is determined (S17). If the estimated value G is not within the management standard range (NO in S17), the water-soluble lubricating oil is diagnosed as degraded, and the diagnosis code 3 is flagged because the kinematic viscosity at 40°C does not fall within the appropriate range even if the water content is adjusted to an appropriate value by adding water to the water-soluble lubricating oil (S18). Then, the process proceeds to the next step. If the estimated value G is within the management standard range (for example, if it is above the lower limit of the management standard) (YES in S17), the process directly proceeds to the next step. The appropriate water content is a given water content that falls within the management standard range. The estimated value G may be presumed by applying the rate of change (proportionality coefficient) of the standard correlation between water content and kinematic viscosity to the detected water content and kinematic viscosity at 40°C, or the estimated value G may be presumed from the detected data of the water content and kinematic viscosity at 40°C and the amount of deviation of the standard correlation between water content and kinematic viscosity.

The standard correlation between water content and kinematic viscosity (hereafter referred to as the standard correlation) for water-soluble lubricating oil that has not degraded (for example, water-soluble lubricating oil before use) is obtained in advance and stored in the diagnosis section 62. The correlation between water content and kinematic viscosity of degraded water-soluble lubricating oil varies from the standard correlation. For example, even if the water content is kept constant, the kinematic viscosity of degraded water-soluble lubricating oil is lower than that of non-degraded water-soluble lubricating oil.

Depending on the degree of degradation, if water is added to the water-soluble lubricating oil in order to optimize the water content, the kinematic viscosity of the water-soluble lubricating oil may be lower than that calculated based on the standard correlation and thus may be outside the management standard. In other words, even if the kinematic viscosity at 40°C is within the management standard range before water is added to the water-soluble lubricating oil, the kinematic viscosity at 40°C may fall outside the management standard range after water is added. In this case, the management standard for both water content and kinematic viscosity would not be satisfied. Thus, diagnosing water-soluble lubricating oil based on the correlation between water content and kinematic viscosity is useful for properly managing water-soluble lubricating oil and eliminating unnecessary adjustment work. This also contributes to a reduction in the amount of liquid waste.

Next, whether the cleanliness is within the management standard value is determined (S19). If the cleanliness is outside the management standard value (NO in S19), the diagnostic code 4 is flagged (S20). Then, the process proceeds to the next step. If the cleanliness is within the management standard value (YES in S19), the process directly proceeds to the next step.

Next, whether the hydrogen ion concentration (pH) is within the management standard value is determined (S21). If the pH value is outside the management standard value (NO in S21), the diagnostic code 5 is flagged (S22). Then, the process proceeds to the next step. If the pH value is within the management standard value (YES in S21), the process directly proceeds to the next step.

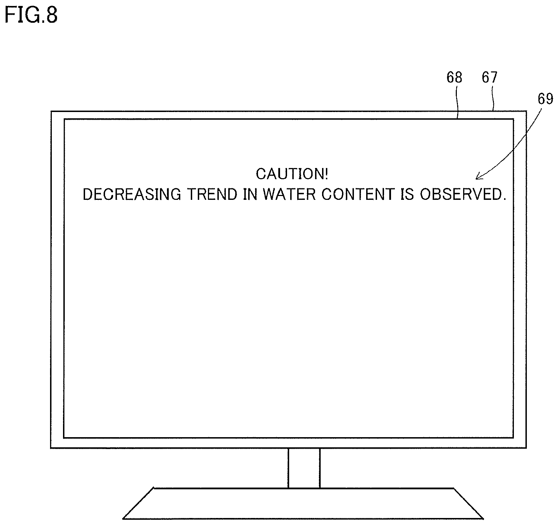

Then, notations are made according to the diagnosis codes 1, 2, 3, 4, and 5 (S23). FIG. 8 is a schematic diagram illustrating an example of a display screen 68 on a display 67 displaying the diagnosis results of the monitoring and diagnosing apparatus 11. Referring to FIG. 8, when the diagnosis results of the diagnosis code 1 are displayed, the display 67 displays the characters 69: "CAUTION! DECREASING TREND IN WATER CONTENT IS OBSERVED.", for example, on the display screen 68. When the diagnosis results of the diagnosis code 2 are displayed, the display 67 displays the characters: "CAUTION! DETERIORATION TREND IN WATER-SOLUBLE LUBRICATING OIL QUALITY IS OBSERVED.", for example. In this way, the warning notification section 65 notifies a warning if the diagnosis section 62 determines that the detected data is outside the prescribed numerical range. In this way, it becomes easier to make this warning known when it is necessary to issue a warning as a serious consequence of the diagnostic results.

According to the monitoring and diagnosing apparatus 11 in the first embodiment, in a working machine 13 that uses water-soluble lubricating oil, the properties of the water-soluble lubricating oil are detected by the detection unit 16, and the properties of the water-soluble lubricating oil are diagnosed by the diagnosis section 62. This would reduce the need for periodic sampling and analysis of the water-soluble lubricating oil by human hands in terms of maintenance and long-term control. Thus, according to the above monitoring and diagnosing apparatus 11, the properties of water-soluble lubricating oil can be efficiently monitored, and the properties of water-soluble lubricating oil can be diagnosed.

In the present embodiment, the detection unit 16 detects the water content of the water-soluble lubricating oil, the temperature of the water-soluble lubricating oil, the density of the water-soluble lubricating oil, the viscosity of the water-soluble lubricating oil, the hydrogen ion concentration of the water-soluble lubricating oil, the cleanliness of the water-soluble lubricating oil, and the appearance of the water-soluble lubricating oil. Thus, the properties of the water-soluble lubricating oil can be checked by detecting the above parameters, and the properties of the water-soluble lubricating oil can be diagnosed in a more detailed and comprehensive manner.

In the present embodiment, the detection unit 16 detects the refractive index of the water-soluble lubricating oil, and derives the water content of the water-soluble lubricating oil based on the detected refractive index of the water-soluble lubricating oil to detect the water content of the water-soluble lubricating oil. Thus, the water content of water-soluble lubricating oils, an important parameter in water-soluble lubricating oils, can be detected more accurately and simply. Thus, more efficient monitoring and diagnosis of the properties of water-soluble lubricating oil can be performed.

Here, the system in this embodiment includes a water tank 41 as a water storage unit for storing water and a chemical liquid tank 42 as a chemical liquid storage unit for storing the chemical liquid that constitutes the water-soluble lubricating oil. The first control unit may control the supply of at least either one of the water and the chemical liquid from the water tank 41 and chemical liquid tank 42 to the water-soluble lubricating oil according to the diagnosis results by the diagnosis section 62. In this way, adjusting one of the properties of water-soluble lubricating oil, such as the water content, pH, or kinetic viscosity of water-soluble lubricating oil, becomes easier without manual intervention. Thus, such a monitoring and diagnosing system 12 is a more user-desired monitoring and diagnosing system. If water is added in S16 and S17 until the water content reaches the appropriate water content, the amount of water added may be changed when it is determined that the kinematic viscosity at 40°C does not fall within the management standard range. For example, if the diagnosis section 62 determines that the kinematic viscosity at 40°C would be brought within the management standard range when the water content is a provisional target value that falls within the management standard range, although it is not an appropriate water content, the supply control section 63 may control the solenoid valve 43 to add water so that the water content will become the provisional target value. Chemical liquids such as thickeners to raise the kinematic viscosity may also be added. For example, the supply control section 63 may add water so that the water content be at an appropriate water content and add a chemical liquid so that the kinematic viscosity at 40°C be within an appropriate management range. Similarly, the amount of the chemical liquid added may be determined based on the estimated value G.

In the present embodiment, the monitoring and diagnosing system 12 including the monitoring and diagnosing apparatus 11 includes a data storage unit 66 that accumulates diagnostic data that are diagnostic results by the diagnosis section 62 and a second control unit 19 that controls the diagnostic data accumulated in the data storage unit 66 to be reflected in the next diagnosis by the diagnosis section 62. Thus, the diagnosis can be made more appropriately based on the accumulated diagnostic data. The data storage unit 66 may also accumulate detected data. The diagnosis section may diagnose the properties of the water-soluble lubricating oil based on the diagnostic data and/or detected data accumulated in the data storage unit. Specifically, for example, the rate of change in the properties of the water-soluble lubricating oil may be calculated based on the diagnosis data and/or detected data stored in the data storage unit 66, then the calculated rate of change may be used to predict the replacement time or the like of the water-soluble lubricating oil and inform the user by displaying the prediction results on the display 67, and the like.

### (Other Embodiment)

In the above embodiment, the detection unit may detect at least one of the water content of the water-soluble lubricating oil, the temperature of the water-soluble lubricating oil, the density of the water-soluble lubricating oil, the viscosity of the water-soluble lubricating oil, the hydrogen ion concentration of the water-soluble lubricating oil, the cleanliness of the water-soluble lubricating oil, and the appearance of the water-soluble lubricating oil. In this way, the property of the water-soluble lubricating oil can be checked by detecting the above parameters, and the property of the water-soluble lubricating oil can be diagnosed in a more detailed and comprehensive manner.

In the above embodiment, the system may include at least either one of a water storage unit that stores water and a chemical liquid storage unit that stores a chemical constituting the water-soluble lubricating oil. The first control unit may control the supply of at least either one of the water and the chemical liquid from at least either one of the water storage unit and the chemical liquid storage unit to the water-soluble lubricating oil in accordance with the diagnosis results by the diagnosis section. In this way, adjusting one of the properties of water-soluble lubricating oil, such as the water content of water-soluble lubricating oil, becomes easier without manual intervention. Thus, a more user-desired monitoring and diagnosing system can be provided.

It should be noted that the warning notification section may notify a warning by at least one of a displayed warning, such as an alert on a screen, a voice warning, or a warning via a network such as e-mails.

In addition, the monitoring and diagnosing method according to the present invention monitors the property of water-soluble lubricating oil used in working machines and diagnoses the property of the water-soluble lubricating oil. The monitoring and diagnosing method includes the steps of: acquiring water-soluble lubricating oil from a working machine; detecting, after the step of acquiring the water-soluble lubricating oil, the property of the acquired water-soluble lubricating oil; acquiring, after the step of detecting the property of the water-soluble lubricating oil, data of the detected the property of the water-soluble lubricating oil; and diagnosing, after the step of acquiring the data of the property of the water-soluble lubricating oil, the property of the water-soluble lubricating oil based on the acquired data on the property of the water-soluble lubricating oil.

According to such a monitoring and diagnosing method, the property of water-soluble lubricating oil can be efficiently monitored, and the property of water-soluble lubricating oil can be diagnosed.

It should be understood that the embodiments disclosed here are illustrative in all respects and not restrictive in any respect. The scope of the invention is defined by the claims and is intended to include the interpretations that are equivalent to the claims and all modifications within the scope of the claims.

### [Reference Signs List]

11 Monitoring and diagnosing apparatus, 12 Monitoring and diagnosing system, 13 Working machine, 14 Oil tank, 15 First control unit, 16 Detection unit, 17 Water and chemical liquid supply unit, 18 Filtration unit, 19 Second control unit, 21 Refractive index sensor, 22 Viscosity sensor, 23 Density sensor, 24 Temperature sensor, 25 Cleanliness sensor, 26 Hydrogen ion concentration detection sensor, 27 Camera, 31, 51 Motor, 32, 52 Feed pump, 33, 53, 54 Filter, 34, 45, 46, 55, 56 Pipe, 41 Water tank, 42 Chemical liquid tank, 43, 44 Solenoid valve, 61 Detected data acquisition section, 62 Diagnosis section, 63 Supply control section, 64 Output control section, 65 Warning notification section, 66 Data storage unit, 67 Display, 68 Display screen, 69 Characters.

## Claims

1. A monitoring and diagnosing apparatus that monitors a property of water-soluble lubricating oil circulating in a working machine and diagnoses the property of the water-soluble lubricating oil, the apparatus comprising:
a first control unit that controls the monitoring and diagnosing apparatus and
a detection unit that detects the property of the water-soluble lubricating oil circulating in the working machine,
the first control unit including
a detected data acquisition section that acquires detected data detected by the detection unit and
a diagnosis section that diagnoses the property of the water-soluble lubricating oil based on the detected data acquired by the detected data acquisition section.

2. The monitoring and diagnosing apparatus according to claim 1, wherein the detection unit detects at least one of a water content of the water-soluble lubricating oil, a temperature of the water-soluble lubricating oil, a density of the water-soluble lubricating oil, a viscosity of the water-soluble lubricating oil, a hydrogen ion concentration of the water-soluble lubricating oil, cleanliness of the water-soluble lubricating oil, and appearance of the water-soluble lubricating oil.

3. The monitoring and diagnosing apparatus according to claim 1 or claim 2, wherein the detection unit detects a refractive index of the water-soluble lubricating oil, and derives water content of the water-soluble lubricating oil based on the detected refractive index of the water-soluble lubricating oil to detect water content of the water-soluble lubricating oil.

4. The monitoring and diagnosing apparatus according to any one of claims 1 to 3, wherein the detection unit detects a viscosity of the water-soluble lubricating oil, a density of the water-soluble lubricating oil, and a temperature of the water-soluble lubricating oil, and derives a kinematic viscosity of the water-soluble lubricating oil at a given temperature.

5. The monitoring and diagnosing apparatus according to any one of claims 1 to 3, wherein
the detection unit detects a water content of the water-soluble lubricating oil, a viscosity of the water-soluble lubricating oil, a density of the water-soluble lubricating oil, and a temperature of the water-soluble lubricating oil, and calculates a kinematic viscosity at a given temperature using the viscosity, the density, and the temperature, and
the diagnosis section estimates a hypothetical kinematic viscosity of the water-soluble lubricating oil at a given temperature when the water-soluble lubricating oil is at an appropriate water content based on the water content detected by the detection unit, the kinematic viscosity at the given temperature, and a standard correlation between the appropriate water content and the kinematic viscosity at the given temperature stored in advance, and determines a degree of degradation of the water-soluble lubricating oil.

6. The monitoring and diagnosing apparatus according to any one of claims 1 to 5, wherein
the diagnosis section determines whether the detected data is outside a prescribed numerical range, and
the first control unit further includes a warning notification section that notifies a warning if the diagnosis section determines that the detected data is outside the prescribed numerical range.

7. A monitoring and diagnosing system including the monitoring and diagnosing apparatus of any one of claims 1 to 6, the monitoring and diagnosing system comprising:
a data storage unit that accumulates diagnostic data that are diagnostic results by the diagnosis section and/or the detected data and
a second control unit that controls the diagnostic data and/or detected data accumulated in the data storage unit to be reflected in the next diagnosis by the diagnosis section, wherein
the diagnosis section diagnoses the property of the water-soluble lubricating oil based on the diagnostic data and/or detected data accumulated in the data storage unit.

8. The monitoring and diagnosing system according to claim 7, the system comprising at least either one of a water storage unit that stores water and a chemical liquid storage unit that stores a chemical liquid constituting the water-soluble lubricating oil, wherein
the first control unit includes a supply control section that controls the supply of at least either one of the water and the chemical liquid from at least one of the water storage unit and the chemical liquid storage unit to the water-soluble lubricating oil in accordance with the diagnosis results by the diagnosis section.

9. The monitoring and diagnosing system according to claim 8, wherein the supply control section determines the supply amount of at least either one of the water and the chemical liquid to be added to the water-soluble lubricating oil based on the hypothetical kinematic viscosity estimated by the diagnosis section.

10. A monitoring and diagnosing method that monitors a property of water-soluble lubricating oil circulating in a working machine and diagnoses the property of the water-soluble lubricating oil, the method comprising the steps of:
detecting the property of the water-soluble lubricating oil circulating in the working machine,
acquiring, after the step of detecting the property of the water-soluble lubricating oil, detected data of the detected the property of the water-soluble lubricating oil, and
diagnosing, after the step of acquiring detected data, the property of the water-soluble lubricating oil based on the acquired detected data.
